(19) Europäisches Patentamt
European Patent Office
Office européen des brevets

(11) EP 4 044 996 B1

(12) **EUROPEAN PATENT SPECIFICATION**

(45) Date of publication and mention of the grant of the patent:
**06.12.2023 Bulletin 2023/49**

(21) Application number: **20788817.3**

(22) Date of filing: **13.10.2020**

(51) International Patent Classification (IPC):
***A61K 8/34*** *(2006.01)* ***A61K 8/35*** *(2006.01)*
***A61K 8/60*** *(2006.01)* ***A61K 8/63*** *(2006.01)*
***A61Q 11/00*** *(2006.01)*

(52) Cooperative Patent Classification (CPC):
**A61Q 11/00; A61K 8/347; A61K 8/35; A61K 8/602; A61K 8/63**

(86) International application number:
**PCT/EP2020/078793**

(87) International publication number:
**WO 2021/074158 (22.04.2021 Gazette 2021/16)**

(54) **ORAL CARE COMPOSITION**

MUNDPFLEGEZUSAMMENSETZUNG

COMPOSITION D'HYGIÈNE BUCCALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2019 EP 19382897**

(43) Date of publication of application:
**24.08.2022 Bulletin 2022/34**

(73) Proprietor: **Lacer, S.A.**
**08290 Cerdanyola del Vallès Barcelona (ES)**

(72) Inventors:
- **MATA MOLINER, Montserrat**
**08290 Cerdanyola del Vallès, Barcelona (ES)**
- **HUARTE FOURNIER, Elena**
**08290 Cerdanyola del Vallès, Barcelona (ES)**
- **DEL CASTILLO NIETO, Juan Carlos**
**08290 Cerdanyola del Vallès, Barcelona (ES)**
- **MIRA OTAL, Javier**
**08290 Cerdanyola del Vallès, Barcelona (ES)**
- **WIEDEMANN, Ralf**
**08290 Cerdanyola del Vallès, Barcelona (ES)**

(74) Representative: **ABG Intellectual Property Law, S.L.**
**Avenida de Burgos, 16D**
**Edificio Euromor**
**28036 Madrid (ES)**

(56) References cited:
- **DATABASE GNPD [Online] MINTEL; 1 August 2019 (2019-08-01), anonymous: "Day & Night Care Advanced Protection Toothpaste with Swiss Herb & Zinc Mineral", XP055675318, retrieved from www.gnpd.com Database accession no. 6755931**
- **DATABASE GNPD [Online] MINTEL; 25 September 2019 (2019-09-25), anonymous: "Gentle Whitening Toothpaste", XP055675314, retrieved from www.gnpd.com Database accession no. 6891557**
- **PARK ET AL.: "Clinical Study on Effects of Dentifrice Containing 0.05% Isopropyl Methylphenol (IPMP) and 0.05% Dipotassium Glycyrrhizinate (GK2) on Gingival Conditions", INTERNATIONAL JOURNAL OF CLINICAL PREVENTIVE DENTISTRY, vol. 6, no. 2 June 2010 (2010-06), pages 55-61, XP002798338, Retrieved from the Internet: URL:http://www.ijcpd.org/journal/view.html ?uid =137&page=&sort=&scale=10&all_k=&s_t=& s_a=&s_k=&s_v=6&s_n=2&spage=&pn=search &yea r=&vmd=Full#body01**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 044 996 B1

- MITSOULIS ET AL: "Annular extrudate swell of pseudoplastic and viscoplastic fluids", JOURNAL OF NON-NEWTONIAN FLUID MECHANICS, ELSEVIER, NL, vol. 141, no. 2-3, 19 January 2007 (2007-01-19), pages 138-147, XP005836024, ISSN: 0377-0257, DOI: 10.1016/J.JNNFM.2006.10.004

## Description

### FIELD OF THE INVENTION

The invention relates to the field of oral care compositions, in particular to mouthwashes, tooth gels and toothpastes comprising o-cymen-5-ol, dipotassium glycyrrhizinate and 4-hydroxyacetophenone.

### BACKGROUND OF THE INVENTION

Oral hygiene is the practice of keeping the mouth clean, and is considered to be the best means of prevention of cavities (dental caries), gingivitis, periodontitis, and other dental and oral disorders such as bad breath (halitosis). Oral hygiene is necessary for all persons to maintain the health of their teeth and mouth. There are three key steps to regular dental health and maintenance: brushing, flossing and rinsing with a mouthwash. Teeth brushing involves the use of a toothpaste in conjunction with a toothbrush to help remove food debris and dental plaque. Mouthwashes are used for cleaning and freshening the mouth and for oral hygiene. Antiseptic and anti-plaque compounds are present in mouthwashes and toothpaste to rinses kill germs that cause plaque, gingivitis, and bad breath.

Glycyrrhizin compounds have been reported as having anti-inflammatory activity which helps in preventing gingivitis and periodontitis [Asl, M. N. et al, Phytother. Res., 2008, 22, 709-724].

It has also been reported that isopropylmethylphenols penetrate deep-inside plaque and show high bactericidal effect [Park, Y.-D. et al., Int. J. Clin. Prev. Dent., 2010, 6(2), 55-61]. Further, isopropylmethylphenols do also reduce the breath concentrations of volatile sulphur compounds (VSC), which are associated with halitosis [Cho et al., Int J Oral Health, 2009, 5, 37-47].

Combinations of these ingredients, i.e. glycyrrhizin compounds and isopropylmethylphenols have been disclosed as being particularly advantageous. In particular, a study conducted by Cho et al. [Int J Oral Health, 2009, 5, 37-47] concluded that a mouthwash comprising these ingredients was safe and effective in terms of reduction of periodontal pocket depth and gingival crevicular fluid, which are markers of periodontal disease, and also in terms of effective inhibition of oral microorganisms. In a clinical study reported by Park et al. [Int. J. Clin. Prev. Dent., 2010, 6(2), 55-61], the effect of dentifrice containing isopropylmethylphenol and dipotassium glycyrrhizinate on gingival conditions showed that the combination of these compounds reduced halitosis, plaque index, gingival crevicular fluid, periodontal pocket depth and gingival index. The authors of the report concluded that the combination of isopropylmethylphenol and dipotassium glycyrrhizinate seemed to show a synergistic effect.

Therefore, the use of isopropylmethylphenols and glycyrrhizin compounds in oral care products is highly desirable, in particular to help control the development of dental plaque, calculus formation, halitosis, gingivitis and periodontitis.

For the manufacture of oral care products, knowledge of fluid rheology is important for process equipment such as pipelines, pumps, and mixers, which are commonly used in manufacturing processes.

According to their rheological behavior, fluids can be classified as Newtonian and non-Newtonian. Newtonian fluids are those exhibiting a linear relationship between shear rate and shear stress. In these fluids, the slope $\eta$ (viscosity) is constant and therefore, the viscosity of a Newtonian fluids is independent of shear rate. On the other hand, in non-Newtonian fluids, the viscosity is dependent on the shear rate. Non-Newtonian fluids may exhibit either a shear thinning or a shear thickening behaviour and, in some cases, these fluids also exhibit a yield stress, i.e. a stress level which must be overcome before the fluid begins to flow. Most fluid compositions, such as oral care compositions and intermediate fluid compositions used in their manufacturing processes, show a non-Newtonian behavior. Non-Newtonian fluids can be classified as plastics fluids, dilatant fluids and pseudoplastic fluids. In plastic fluids, a critical level of stress (yield stress) must be attained in order to initiate flow. Below this critical stress, $\tau$, the material behaves as a solid, absorbing the stress energy without flowing. Once the threshold of critical stress has been reached, the material yields to flow, hence the term, yield stress. In pseudoplastic fluids the viscosity decreases as shear rate increases, but these fluids do not have a yield stress, i.e. they flow instantaneously upon application of stress. Dilatant fluids exhibit shear thickening properties, i.e. the fluid viscosity increases as the shear rate increases.

Rheological studies are used to evaluate the properties of fluids. Flow curve models are used to characterize the forms of flow behavior. Models describing pseudoplastic behaviour include the power-law, Carreau, Cross, etc. Models describing plastic behaviour include the Bingham, Herschel-Bulkley and Casson [Mitsoulis, J. Non-Newtonian FluidMech., 2007, 141, 138-147].

In industrial manufacturing processes, pseudoplastic fluids are preferred to plastic fluids since there is no stress level that must be overcome before the fluid begins to flow. When a yield stress has to be overcome, as in plastic fluids, pumps and mixers are negatively affected, and pipelines may get clogged.

Thus, there is a need for alternative oral care products comprising isopropylmethylphenols and glycyrrhizin compounds and having suitable rheological properties for the manufacturing process, i.e. having a pseudoplastic fluid

behavior.

## SUMMARY OF THE INVENTION

The present inventors have surprisingly found that compositions comprising isopropylmethylphenols such as o-cymen-5-ol, and/or glycyrrhizin compounds, such as dipotassium glycyrrhizinate, do not have desirable rheological properties for manufacturing processes since they behave has plastic fluids. Unexpectedly, the addition of hydroxyacetophenone to a composition comprising a isopropylmethylphenol and a glycyrrhizin compound modifies the rheological behavior of the composition to that of a pseudoplastic fluid, which is preferred in the process of manufacture of oral care compositions. The compositions of the invention are also advantageous due to their low cytotoxicity, antioxidative effects, anti-inflammatory effects, wound healing properties, microbiological profiles, cleaning properties, plaque penetration and reduction properties, their properties with respect to their stability, in particular against light, temperature, or external forces such as gravity forces.

Thus, in a first aspect, the present invention relates to an oral care composition comprising:

- 0.005 to 1 wt% of o-cymen-5-ol,
- 0.005 to 1.5 wt% of dipotassium glycyrrhizinate,
- 0.05 to 5 wt% of 4-hydroxyacetophenone, and
- at least 15 wt% of water.

In a second aspect, the present invention relates to an oral care composition as defined in the first aspect for use in the treatment and/or prevention of dental plaque formation, caries formation, calculus formation, halitosis, gingivitis, periodontitis, peri-implant mucositis and/or periimplantitis.

## DETAILED DESCRIPTION OF THE INVENTION

Oral care composition according to the claims are disclosed herein comprising

- an isopropylmethylphenol compound,
- a glycyrrhizin derivative selected from the group consisting of glycyrrhizinic acid, glycyrrhetinic acid, salts thereof, and mixtures thereof,
- a hydroxyacetophenone, and
- water.

The expression "oral care" compositions, means that the compositions are intended for topical use in the mouth, so any component present in the composition needs to be orally acceptable, that is, safe for topical use in the mouth in the amounts and concentrations provided.

The term "isopropylmethylphenol compound" refers to compounds having a phenyl ring substituted by one hydroxyl group, one methyl group and one isopropyl group, as represented by formula (I).

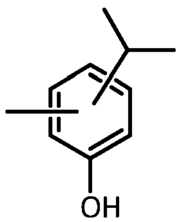

(I)

Examples of isopropylmethyl phenol compounds are o-cymen-5-ol (or 4-isopropyl-3-methylphenol of formula Ia), thymol (or 2-isopropyl-5-methylphenol of formula Ib) and 3-isopropyl-5-methylphenol (of formula Ic), whose structures are depicted below.

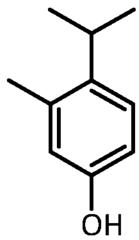

*o*-cymen-5-ol (Ia)

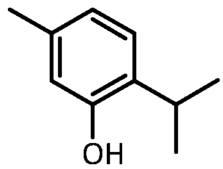

thymol (Ib)

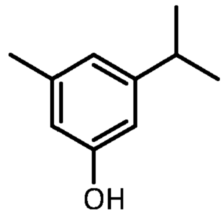

3-isopropyl-5-methylphenol (Ic)

Oral care compositions described herein may comprise one or more (such as 2 or 3) isopropylmethylphenol compounds, preferably one.

Isopropylmethylphenol compounds may be present in the oral care compositions described herein in a concentration of from 0.005 to 1 wt% with respect to the total weight of the composition, preferably from 0.01 to 0.5 wt%, more preferably from 0.02 to 0.1 wt%.

In the compositions of the present invention, the isopropylphenylphenol compound is *o*-cymen-5-ol.

In the compositions of the present invention, o-cymen-5-ol is present in a concentration of from 0.005 to 1 wt% with respect to the total weight of the composition, preferably from 0.01 to 0.5 wt%, more preferably from 0.02 to 0.1 wt%.

The glycyrrhizin derivative present in the oral care compositions described herein may be selected from the group consisting of glycyrrhizinic acid, glycyrrhetinic acid, salts thereof, and mixtures thereof. These compounds are widely applied as a natural sweeteners, and utilized in cosmetics and pharmaceuticals. They can be isolated from the roots and stolon of perennial plants of *Glycyrrhiza* species, such as *Glycyrrhiza glabra.*

"Glycyrrhizinic acid" or "glycyrrhizic acid" has the chemical structure depicted below.

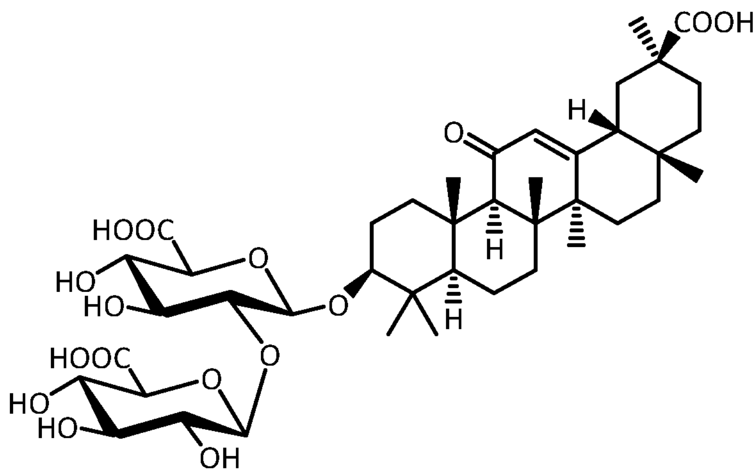

(II)

Glycyrrhizinic acid has three acid groups which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhizinic acid are potassium, sodium and ammonium. Examples of glycyrrhizinic acid salts are dipotassium glycyrrhizinate, diammonium glycyrrhizinate, disodium glycyrrhizinate, monoammonium glycyrrhizinate, monosodium glycyrrhizinate, and monopotassium glycyrrhizinate, preferably dipotassium glycyrrhizinate.

"Glycyrrhetinic acid" or "enoxolone" has the chemical structure depicted below.

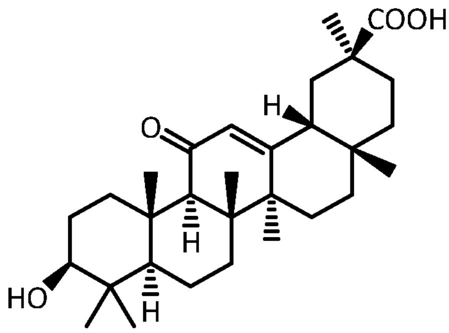

(III)

Glycyrrhetinic acid has one acid group which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhetinic acid are potassium, sodium and ammonium. Examples of glycyrrhetinic acid salts are potassium glycyrrhetinate, ammonium glycyrrhetinate, and sodium glycyrrhetinate.

The glycyrrhizin derivative in the oral care compositions described herein may be glycyrrhizinic acid or a salt thereof, more preferably dipotassium glycyrrhizinate.

The glycyrrhizin derivative may be present in the oral care compositions described herein in a concentration of from 0.005 to 1.5 wt% with respect to the total weight of the composition, preferably from 0.01 to 1.5 wt%, more preferably from 0.02 to 1 wt%, more preferably from 0.02 to 0.5 wt%.

In the oral care compositions of the present invention, the glycyrrhizin derivative is dipotassium glycyrrhizinate.

In the oral care compositions of the present invention, dipotassium glycyrrhizinate is present in a concentration of from 0.005 to 1.5 wt% with respect to the total weight of the composition, preferably from 0.01 to 1.5 wt%, more preferably from 0.02 to 1 wt%, more preferably from 0.02 to 0.5 wt%.

The term hydroxyacetophenone refers to a compound of formula (IV).

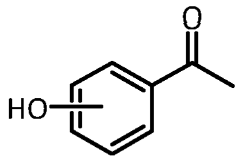

(IV)

Examples of hydroxyacetophenones are 2-hydroxyacetophenone, 3-hydroxyacetophenone and 4-hydroxyaceotphenone.

The oral care compositions described herein may comprise one or more (such as 2 or 3) hydroxyacetophenones, preferably one.

The hydroxyacetophenone may be present in the oral care compositions described herein in a concentration of from 0.05 to 5 wt% with respect to the total weight of the composition, preferably from 0.05 to 1.5 wt%, more preferably from 0.05 to 1 wt%, even more preferably from 0.1 to 1 wt%.

In the oral care compositions of the present invention, the hydroxyacetophenone is 4-hydroxyacetophenone.

In the oral care compositions of the present invention, 4-hydroxyacetophenone is present in a concentration of from 0.05 to 5 wt% with respect to the total weight of the composition, preferably from 0.05 to 1.5 wt%, more preferably from 0.05 to 1 wt%, even more preferably from 0.1 to 1 wt%.

Thus, the oral compositions according to the present invention comprises:

- 0.005 to 1 wt% of o-cymen-5-ol,
- 0.005 to 1.5 wt% of dipotassium glycyrrhizinate,
- 0.05 to 5 wt% of 4-hydroxyacetophenone, and
- at least 15 wt% of water, wherein the wt% are expressed with respect to the total weight of the composition.

In the oral care compositions described herein, the weight ratio of the hydroxyacetophenone to the glyzyrrhizin derivative is from 15:1 to 0.5:1, preferably from 15:1 to 1:1, more preferably from 10:1 to about 1:1, even more preferably

about 1:1.

In the oral care compositions described herein, the weight ratio of the hydroxyacetophenone to the isopropylmethylphenol compound is from 10:1 to 0.5:1, preferably from 10:1 to 1:1, more preferably from 5:1 to about 1:1, even more preferably about 1:1.

Thus, in a preferred embodiment of the oral care compositions according to the present invention, the weight ratio of 4-hydroxyacetophenone to dipotassium glycyrrhizinate is from 15:1 to 0.5:1, preferably from 15:1 to 1:1, more preferably from 10:1 to about 1:1, even more preferably about 1:1.

Thus, in another preferred embodiment of the oral care compositions according to the present invention, the weight ratio of 4-hydroxyacetophenone to the o-cymen-5-ol is from 10:1 to 0.5:1, preferably from 10:1 to 1:1, more preferably from 5:1 to about 1:1, even more preferably about 1:1.

The oral care compositions described herein, in particular those of the present invention, also comprise water. Preferably, water is present in an amount of at least 15 wt%, at least 20 wt%, at least 25 wt%, at least 30 wt%, at least 40 wt%, at least 50 wt%, at least 60 wt%, at least 70 wt%, at least 75 wt%, or at least 80 wt%, wherein the wt% is expressed with respect to the total weight of the composition.

Preferably, the oral care compositions described herein, in particular those of the present invention, also comprise one or more ingredients selected from the group consisting of fluoride ion sources, surfactants, co-solvents, humectants, sweeteners, binders, mouth-feel agents, abrasives, desensitizing agents, other oral care active ingredients, pH modifying agents, chelating agents, preservatives, flavoring agents, and colorants.

Fluoride ion sources include sodium fluoride, potassium fluoride, stannous fluoride, potassium monofluorophosphate, sodium monofluorophosphate, ammonium monofluorophosphate, sodium fluorosilicate, ammonium fluorosilicate, amine fluoride such as (N'-octadecyltrimethylendiamine-N,N,N'-tris(2-ethanol)-dihydrofluoride), ammonium fluoride, and combinations thereof. Preferably, the oral care compositions described herein, in particular those of the present invention, may also contain a source of fluoride ions or fluorine-providing ingredient in amounts sufficient to supply 50 to 5000 ppm fluoride ion, e.g., from 100 to 1000 ppm fluoride ion. Fluoride ion sources may be added to the compositions described herein, in particular to those of the present invention, at a level in the range of 0.001 wt% to 10 wt%, e.g., from 0.003 wt% to 5 wt%, or from 0.01 wt% to 1.2 wt%, wherein the wt% is expressed with respect to the total weight of the composition. However, it is to be understood that the weights of fluoride salts to provide the appropriate level of fluoride ion will obviously vary based on the weight of the counter ion in the salt, and one of skill in the art may readily determine such amounts. Preferred fluoride ion sources are sodium fluoride and sodium monofluorophsphate.

The oral care compositions described herein, in particular those of the present invention, may optionally comprise a surfactant. The term "surfactant" refers to compounds that are capable of lowering the surface tension. Suitable surfactants include non-ionic, anionic, cationic and amphoteric surfactants. Any orally acceptable surfactant can be used.

Examples of non-ionic surfactants are block copolymers such as ethylene oxide and propylene oxide block copolymers (e.g. poloxamers), ethoxylated hydrogenated castor oils, polyoxyethylene sorbitan esters (e.g. polysorbates), fatty alcohol ethoxylates, alkylphenol ethoxylates, tertiary amine oxides, tertiary phosphine oxides, dialkylsulfoxides and the like. In a particular embodiment, the non-ionic surfactant is selected from ethylene oxide and propylene oxide block copolymers (e.g. poloxamers), ethoxylated hydrogenated castor oils, and mixtures thereof; preferably poloxamer 407, PEG 40 hydrogenated castor oil and mixtures thereof.

Examples of anionic surfactants are alkyl and alkenyl sulfates and their alkyl-ether derivatives, sarcosinates, isethionates and taurates, such as sodium lauryl sulfate, sodium coconut monoglyceride sulfonate, sodium lauryl sarcosinate, sodium lauryl isethionate, sodium laureth carboxylate, salts of $C_8$-$_{20}$alkyl sulfates (such as sodium $C_{14}$-$C_{16}$ alkyl suflates), salts of $C_8$-$_{20}$alkenyl sulfates (such as sodium $C_{14}$-$C_{16}$ alkenyl sulfates), and sodium dodecyl benzenesulfonate; preferably sodium lauryl sarcosinate and sodium salts of $C_8$-$_{20}$alkyl sulfates (such as sodium $C_{14}$-$C_{16}$ alkyl suflates) and sodiumc salts of $C_8$-$_{20}$alkenyl sulfates (such as sodium $C_{14}$-$C_{16}$ alkenyl sulfates).

Examples of cationic surfactants are lauryl trimethylammonium chloride, cetyl pyridinium chloride, cetyl trimethylammonium bromide, diisobutylphenoxyethyl dimethylbenzylammonium chloride, coconut alkyltrimethylammonium nitrite, cetyl pyridinium fluoride and mixtures thereof.

Examples of amphoteric surfactants are myristyl betaine, palmityl betaine, lauryl betaine, cetyl betaine, stearyl betaine, lauramidopropyl betaine, cocoamidoethyl betaine, cocoamidopropyl betaine and the like; preferably cocamidopropylbetaine.

Preferred surfactants include polyoxyethylene hydrogenated castor oils, poloxamers (polypropylene glycol-polyethylene glycol block copolymers), salts of $C_8$-$_{20}$alkyl sulfates, salts of $C_8$-$_{20}$alkenyl sulfates, sodium lauryl sarcosinate, and cocoamidopropyl betaine.

One or more surfactants are optionally present in a total amount in the range of from 1 wt% to 70 wt%, wherein the wt% is expressed with respect to the total weight of the composition.

In addition to water, the oral care compositions described herein, in particular those of the present invention, may comprise one or more co-solvents such as glycerin, propylene glycol and/or ethanol. One or more co-solvents are optionally present in a total amount in the range of from 5 wt% to 20 wt%, wherein the wt% is expressed with respect

to the total weight of the composition.

The oral care compositions described herein, in particular those of the present invention, may optionally comprise a humectant, which is a substance that holds and retains moisture. Any orally acceptable humectant can be used, such as sugar alcohols, e.g. sorbitol and, and low molecular weight PEGs, such as PEG40. Most humectants also function as sweeteners. One or more humectants are optionally present in a total amount in the range of from 1 wt% to 70 wt%, wherein the wt% is expressed with respect to the total weight of the composition.

In addition to the humectants described above and to the glycyrrhizin derivatives described above (which are essential components of the invention), which also function as sweeteners (i.e. substances capable of providing a sweet taste), the oral care compositions described herein, in particular those of the present invention, may optionally comprise a sweetener which is not a humectant. Examples of this type of sweeteners include artificial sweeteners such as sucralose, saccharin, acesulfam, neotam, neohesperidin, cyclamate, thaumatin, stevioside. These sweeteners (which are neither humectants not glycyrrhizin derivatives) may be present in amounts in the range of 0.001 wt% to 2 wt%, wherein the wt% is expressed with respect to the total weight of the composition.

The oral care compositions described herein, in particular those of the present invention, may contain a binder, which provides cohesion to the composition. Any conventional binder may be utilized, such as, carboxyvinyl polymers, carrageenans, starches, cellulosic polymers e.g. hydroxyethylcellulose. carboxymethylcellulose (carmellose), hydroxypropyl methyl cellulose and salts thereof (e.g., carmellose sodium), natural gums such as xanthan gum, karaya, gum arabic and tragacanth, and chitosan. One or more binders may be present in a total amount in the range 0.1 wt% to 1.5 wt%, wherein the wt% is expressed with respect to the total weight of the composition. Preferably, the oral care compositions described herein, in particular those of the present invention, comprise xanthan gum, preferably in an amount in the range 0.1 wt% to 1.5 wt%, wherein the wt% is expressed with respect to the total weight of the composition.

Mouth-feel agents that may be present in the oral care compositions described herein, in particular those of the present invention, and refer to materials which impart a desirable texture or other feeling during use of the composition. Such agents include bicarbonate salts, which may impart a clean feel to teeth and gums due to effervescence and release of carbon dioxide. Any orally acceptable bicarbonate can be used, such as alkali metal bicarbonates e.g. sodium and potassium bicarbonates, ammonium bicarbonate, and mixtures thereof. One or more bicarbonate salts are optionally present in a total amount in the range of 0.1 wt% to 5 wt%, wherein the wt% is expressed with respect to the total weight of the composition.

The oral care compositions described herein, in particular those of the present invention, may also include an abrasive, which removes material from the teeth and gum surfaces and aids in mechanical tooth cleaning. The abrasive may be a silica abrasive such silica, sodium metaphosphate, potassium metaphosphate, tricalcium phosphate, dihydrated dicalcium phosphate, aluminum silicate, calcined alumina, bentonite or other siliceous materials, sodium chloride, or combinations thereof. One or more abrasives may be present in a total amount in the range from 10 wt% to 30 wt%, preferably from 15 wt% to 20 wt% %, wherein the wt% is expressed with respect to the total weight of the composition.

The oral care compositions described herein, in particular those of the present invention, may also comprise a desensitizing agents, which treat hypersensitivity by blocking dentin tubules when applied to a tooth. Examples of desensitizing agents are potassium salts such as potassium nitrate, potassium chloride, potassium bicarbonate, potassium citrate, and potassium oxalate. Such agents may be added in effective amounts, e.g., in a total amount typically in the range from 0.01 wt % to 5 wt%, wherein the wt% is expressed with respect to the total weight of the composition.

In addition to the oral care active ingredients mentioned above (such as isopropylmethylphenols, glycyrrhizin derivatives, fluoride ion sources and desenstizing agents), other oral care active ingredients may be present in the compositions described herein, in particular those of the present invention. Said oral care active ingredients may exert any beneficial effect to teeth and gums, such as reducing hypersensitivity of the teeth, reducing plaque accumulation, reducing or inhibiting demineralization and promote remineralization of the teeth, inhibiting microbial biofilm formation in the oral cavity, reducing or inhibiting gingivitis, promoting healing of sores, reducing levels of acid producing bacteria, increasing relative levels of non-cariogenic and/or non-plaque forming bacteria, reducing or inhibiting formation of dental caries, reducing, repairing or inhibiting pre-carious lesions of the enamel, treating, relieving or reducing dry mouth, cleaning the teeth and oral cavity, reducing erosion, whiten teeth, and reducing tartar build-up. Examples of said agents include alkali metal gluconate salts such as sodium gluconate, pantenol, vitamin E or a derivative thereof such as vitamin E acetate, $\alpha$-bisabolol, malic acid, calcium glycerophosphate, etc. It is to be understood that the weights of these active ingredients may vary depending on the particular active ingredient, and one of skill in the art may readily determine such amounts. Typically each oral care active ingredient is optionally present in an amount in the range from 0.001 wt% to 1 wt%%, wherein the wt% is expressed with respect to the total weight of the composition.

The oral care compositions described herein, in particular those of the present invention, may comprise a pH modifying agent, which is a compound that adjust and/or maintain pH. Such agents include acidifying agents to lower pH, basifying agents to raise pH, and buffering agents to control pH within a desired range. For example, one or more compounds selected from acidifying, basifying and buffering agents can be included to provide a pH of 4.5 to 10.5, preferably of 5.5 to 8.5. Any orally acceptable pH modifying agent can be used, including carboxylic, phosphoric and

sulfonic acids and their salts (e.g., monopotassium phosphate, dipotassium phosphate, monosodium phosphate, trisodium phosphate, citric acid, monosodium citrate, disodium citrate, monosodium malate, etc.), alkali metal hydroxides such as sodium hydroxide, carbonates such as sodium carbonate, bicarbonates, sesquicarbonates, borates, silicates, imidazole and the like. One or more pH modifying agents are optionally present in a total amount effective to maintain the composition in an orally acceptable pH range.

The oral care compositions described herein, in particular those of the present invention, may optionally comprise a chelating agent, which forms complexes with metal ions, such as calcium, magnesium and iron. Examples of chelating agents are citric acid, succinic acid, gluconic acid, etidronic acid, galactaric acid, galacturonic acid, glucuronic acid, phytic acid, ethylenediaminetetraacetic acid (EDTA), methylglycine-*N*,*N*-diacetic acid (MGDA), *N*,*N*-Dicarboxymethyl glutamic acid (GLDA), and the respective salts of ammonium, sodium and potassium; poly-acrylic acids, polymers thereof and the respective salts of ammonium, sodium and potassium; and phosphoric acids and polyphosphoric acids and the respective salts of ammonium, sodium and potassium. Preferably, the chelating agent is EDTA or an ammonium, sodium or potassium salt thereof. One or more chelating agents are optionally present in a total amount in the range of from 0.1 to 5 wt%, wherein the wt% is expressed with respect to the total weight of the composition.

Preservatives may also be present in the oral care compositions described herein, in particular those of the present invention. Preservatives inhibit the development of microorganism in the oral care composition. Examples of suitable preservatives are chlorhexidine, triclosan, quaternary ammonium compounds (such as benzalkonium chloride) or parabens (such as methyl or propyl paraben). One or more preservatives are optionally present in a total amount typically in the range from 0.001 wt% to 0.5 wt%, wherein the wt% is expressed with respect to the total weight of the composition.

The oral care compositions described herein, in particular those of the present invention, may optionally comprise a flavoring agent that is added to modify the taste and the aroma of the oral care compositions. Flavoring agents that may be used in the compositions described herein, in particular those of the present invention, include essential oils as well as various flavoring aldehydes, esters, alcohols, and similar materials. Examples of the essential oils include oils of spearmint, peppermint, aniseed, wintergreen, sassafras, clove, sage, eucalyptus, marjoram, cinnamon, lemon, lime, grapefruit, and orange. Also useful are compounds such as menthol, carvone, anethole and vainillin. Of these, the most commonly employed are the oils of peppermint and spearmint. One or more flavoring agents may be present in the oral care composition at a total concentration in the range of 0.01 wt% to 5 wt%, wherein the wt% is expressed with respect to the total weight of the composition.

The oral care compositions described herein, in particular those of the present invention, may comprise at least one colorant, which is added to modify the colour of the compositions. Colorants herein include pigments, dyes, lakes and agents imparting a particular luster or reflectivity such as pearling agents. Any orally acceptable colorant can be used, such as titanium dioxide. One or more colorants are optionally present in a total amount in the range of from 0.0001 wt% to 5 wt%, wherein the wt% is expressed with respect to the total weight of the composition.

The oral care compositions described herein, in particular those of the present invention, may be in the form of a mouthwash, a toothpaste or a tooth gel.

In the context of the present invention, "mouthwash" is equivalent to "oral rinse", "mouth rinse" and "mouth bath" and designate liquid compositions which are suitable for oral hygiene, such as by reducing the microbial load in the oral cavity, in particular the bacterial plaque that causes cavities, gingivitis and bad breath. When using such compositions, they are typically maintained in the mouth passively and/or swished through the teeth by contraction of the perioral muscles and/or movement of the head, and may be gargled, and finally spit out.

In the context of the present disclosure, the term "toothpaste" refers to a semisolid preparation suitable for cleaning the teeth when used with a toothbrush. In the context of the present invention, the term "tooth gel" refers to a gel preparation suitable for cleaning the teeth when used with a toothbrush. Tooth gels are like toothpastes but clear or translucent in appearance. In particular, tooth gels do not comprise titanium dioxide whereas toothpastes comprise titanium dioxide.

Preferably, the oral care composition described herein, in particular that of the present invention, is a mouthwash and comprises water in an amount of at least 70 wt%, at least 75 wt%, or at least 80 wt%; preferably at least 75 wt% of water. The wt% is expressed with respect to the total weight of the composition.

Preferably, the oral care composition described herein, in particular that of the present invention, is a tooth gel or toothpaste and comprises water in an amount of at least 15 wt%, at least 20 wt%, at least 25 wt%, at least 30 wt%; preferably at least 10 wt% of water. The wt% is expressed with respect to the total weight of the composition.

Preferably, the oral care composition described herein, in particular that of the present invention, is a tooth gel or toothpaste and comprises water in an amount of from 15 wt% to 35 wt%, from 20 wt% to 35 wt%, from 25 wt% to 35 wt%, from 30 wt% to 35 wt%; preferably from 10 wt% to 35 wt% of water. The wt% is expressed with respect to the total weight of the composition.

In a particular, the oral care composition described herein is in the form of a mouthwash and comprises:

- 0.01 to 1.5 wt% of a hidroxyacetophenone,
- 0.005 to 1 wt% of an isopropylmethylphenol compound,
- 0.005 to 1.5 wt% of a glycyrrhizin derivative selected from the group consisting of glycyrrhizinic acid, glycyrrhetinic acid, salts thereof, and mixtures thereof,
- at least 70 wt% of water,
- optionally 5 to 10 wt% of propylene glycol,
- optionally 1 to 10 wt% of glycerin, and
- optionally 1 to 5 wt% PEG40 hydrogenated castor oil,

wherein the wt% is expressed with respect to the total weight of the composition.

In a preferred embodiment, the oral care composition of the invention is in the form of a mouthwash and comprises:

- 0.05 to 1.5 wt% of 4-hydroxyacetophenone,
- 0.005 to 1 wt% of o-cymen-5-ol,
- 0.005 to 1.5 wt% of dipotassium glycyrrhizinate,
- at least 70 wt% of water,
- optionally 5 to 10 wt% of propylene glycol,
- optionally 1 to 10 wt% of glycerin, and
- optionally 1 to 5 wt% PEG40 hydrogenated castor oil,

wherein the wt% is expressed with respect to the total weight of the composition.

In a particular, the oral care composition described herein is in the form of a tooth gel or toothpaste, preferably a tooth gel, and comprises:

- 0.01 to 1.5 wt% of a hydroxyacetophenone,
- 0.005 to 1 wt% of an isopropylmethylphenol compound,
- 0.005 to 1.5 wt% of a glycyrrhizin derivative selected from the group consisting of glycyrrhizinic acid, glycyrrhetinic acid, salts thereof, and mixtures thereof,
- 15 to 35 wt% of water,
- 30 to 55 wt% of sorbitol,
- optionally 0.1 to 1.5 wt% xanthan gum,
- optionally 0.5 to 10 wt% of propylene glycol, and
- optionally 1 to 15 wt% of glycerin,

wherein the wt% is expressed with respect to the total weight of the composition.

In a preferred embodiment, the oral care composition of the invention is in the form of a tooth gel or toothpaste, preferably a tooth gel, and comprises:

- 0.05 to 1.5 wt% of 4-hydroxyacetophenone,
- 0.005 to 1 wt% of o-cymen-5-ol,
- 0.005 to 1.5 wt% of dipotassium glycyrrhizinate,
- 15 to 35 wt% of water,
- 30 to 55 wt% of sorbitol,
- optionally 0.1 to 1.5 wt% xanthan gum,
- optionally 0.5 to 10 wt% of propylene glycol, and
- optionally 1 to 15 wt% of glycerin,

wherein the wt% is expressed with respect to the total weight of the composition.

In a particular embodiment, the oral care composition of the invention is in the form of a tooth gel and comprises:

- 0.05 to 1.5 wt% of 4-hydroxyacetophenone,
- 0.005 to 1 wt% of o-cymen-5-ol,
- 0.005 to 1.5 wt% of dipotassium glycyrrhizinate,
- 15 to 35 wt% of water,
- 30 to 55 wt% of sorbitol,
- optionally 0.1 to 1.5 wt% xanthan gum,
- optionally 0.5 to 10 wt% of propylene glycol, and

- optionally 1 to 15 wt% of glycerin,

wherein the wt% is expressed with respect to the total weight of the composition.

In another particular embodiment, the oral care composition of the invention is in the form of a toothpaste and comprises:

- 0.05 to 1.5 wt% of 4-hydroxyacetophenone,
- 0.005 to 1 wt% of o-cymen-5-ol,
- 0.005 to 1.5 wt% of dipotassium glycyrrhizinate,
- 15 to 25 wt% of water,
- 30 to 40 wt% of sorbitol,
- 0.1 to 1.5 wt% of xanthan gum,
- optionally 0.5 to 5 wt% of propylene glycol, and
- optionally 10 to 15 wt% of glycerin,

wherein the wt% is expressed with respect to the total weight of the composition.

The oral care compositions of the invention may be obtained by mixing the above mentioned ingredients.

Uses of the compositions

As explained in the background section, isopropylmethylphenols and/or glycyrrhizin compounds having anti-inflammatory activity which helps in preventing gingivitis and periodontitis [Asl, M. N. et al, Phytother. Res., 2008, 22, 709-724], have high bactericidal effect [Park, Y.-D. et al., Int. J. Clin. Prev. Dent., 2010, 6(2), 55-61], and reduce the breath concentrations of volatile sulphur compounds (VSC), which are associated with halitosis [Cho et al., Int J Oral Health, 2009, 5, 37-47]. The combinations of these compounds has also been reported [Park et al., Int. J. Clin. Prev. Dent., 2010, 6(2), 55-61] to reduce halitosis, plaque index, gingival crevicular fluid, periodontal pocket depth and gingival index.

Accordingly, the oral care compositions described herein are for use in maintaining the health of the oral cavity, in particular for use in the treatment and/or prevention of dental plaque formation, caries formation, calculus formation, halitosis, gingivitis, periodontitis, peri-implant mucositis and/or periimplantitis. The use of an oral care composition as described herein for the manufacture of a medicament for treating and/or preventing dental plaque formation, caries formation, calculus formation, halitosis, gingivitis, periodontitis, peri-implant mucositis and/or periimplantitis is also disclosed herein. Further, a method for the treatment and/or prevention of dental plaque formation, caries formation, calculus formation, halitosis, gingivitis, periodontitis, peri-implant mucositis and/or periimplantitis comprising contacting a surface of the oral cavity with the oral care compositions as described herein is also disclosed.

Thus, a second aspect of the present invention is directed to the oral care compositions of the invention for use in maintaining the health of the oral cavity, in particular for use in the treatment and/or prevention of dental plaque formation, caries formation, calculus formation, halitosis, gingivitis, periodontitis, peri-implant mucositis and/or periimplantitis.

This aspect may also be formulated as the use of an oral care composition of the invention for the manufacture of a medicament for treating and/or preventing dental plaque formation, caries formation, calculus formation, halitosis, gingivitis, periodontitis, peri-implant mucositis and/or periimplantitis.

Alternatively, this aspect may be formulated as a method for the treatment and/or prevention of dental plaque formation, caries formation, calculus formation, halitosis, gingivitis, periodontitis, peri-implant mucositis and/or periimplantitis comprising contacting a surface of the oral cavity with the oral care compositions of the invention.

Alternatively, this aspect may be formulated as the use of the oral care compositions of the invention for the treatment and/or prevention of dental plaque formation, caries formation, calculus formation, halitosis, gingivitis, periodontitis, peri-implant mucositis and/or periimplantitis.

For the above purposes, the aqueous oral compositions described herein, in particular those of the present invention, may be contacted with a surface of the oral cavity. Said surface of the oral cavity may be selected from the group consisting of one or more teeth, surfaces of the gums, surface of the tongue and combinations thereof. For performing said contacting, when the oral care composition of the invention a mouthwash, it is typically held in the oral cavity passively or swilled around the mouth by contraction of the perioral muscles and/or movement of the head, and may be gargled, and finally spit out. In the case of mouthwashes, the amount of composition dosed in the oral cavity to perform the activity is typically from 2-30 ml, preferably from 5-20 ml and most preferable from 10-15 ml. The contacting may be performed for any suitable amount of time, such as for less than thirty seconds, for thirty seconds or more, for about thirty seconds, for about forty seconds, for about one minute or more. For performing the contact with a surface of the oral cavity, when the oral care composition of the invention is a tooth gel or toothpaste, it is typically applied with

a toothbrush and mechanical cleaning of the teeth and/or tongue is performed, preferably for at least two minutes, more preferably for at least three minutes. In the case of tooth gels and toothpastes, the amount of composition dosed to perform the activity is typically from 0.3-4.0 g, preferably from 0.5-2.5 g. The contacting may be carried out at least once daily, preferably at least twice daily, more preferably at least three times daily, such as 1, 2, 3 or 4 times per day. The oral cavity may belong to a human or animal, preferably a human.

The term "treatment", "treating" or "treat", as used herein, refers to means reversing, alleviating, inhibiting the progress of the disease or disorder these terms refer to, or one or more symptoms of such disease or condition.

The term "prevention", "preventing" or "prevent", as used herein, refers to avoiding the appearance of a certain disease or disorder. The prevention can be complete (e.g. the total absence of a disease or disorder). The prevention can also be partial, such that for example the occurrence of a disease or disorder in a subject is less than that which would have occurred without the administration of the oral care composition of the present invention. The prevention also includes reducing the risk of suffering the disease or disorder. In a particular embodiment, the compositions of the invention are for use in prevention.

The term "dental plaque" refers to a fine film comprised of remains of food, bacteria and saliva. If dental plaque is not eliminated regularly, it sticks to the surface of the enamel, attacking tooth structures and producing a series of substances that cause the gum swelling, giving rise to gingivitis (inflammation of the gum), redness and probable gingival bleeding.

The term "calculus" or "tartar" refers to a form of hardened dental plaque. It is caused by precipitation of minerals from saliva and gingival crevicular fluid in plaque on the teeth. This process of precipitation kills the bacterial cells within dental plaque, but the rough and hardened surface that is formed provides an ideal surface for further plaque formation. This leads to calculus buildup, which compromises the health of the gingiva (gums). Calculus can form both along the gumline, where it is referred to as supragingival, and within the narrow sulcus that exists between the teeth and the gingiva, where it is referred to as subgingival.

The term "gingivitis" refers to an inflammation of the gums that gives rise to an increase in sensitivity and irritation and which in many cases is accompanied by bleeding. Its most frequent trigger is dental plaque or biofilm, consisting of a fine film comprised of remains of food, bacteria and saliva. If it is not treated in time, it can evolve to periodontitis (pyorrhoea), and even enter the tooth and damage its bone structure, leading to the irreversible loss of the tooth.

The term "periodontitis" refers to an inflammatory gingival process that spreads to the periodontal ligament which holds and fixes the tooth, and even the alveolar bone, destroying both tissues. Periodontal bags appear, which are an increase in the gap or space between the surface of the tooth and the internal part of the gum. This destructive process spreads until tooth stability is compromised. Due to the loss of fixation, in the end stages the teeth become separated and loose, losing their normal location, and finally the tooth irremediably falls out. *Streptococci*, *spirochetes* and *bacteroides* are found to be the possible pathogens responsible for the disease [Prasanth M., Dent Res J (Isfahan), 2011, 8(2), 85-94; Menendez A. et al., Oral Microbiology Immunology, 2005, 20(1), 31-34].

The term "halitosis" or "bad breath" refers to the presence of unpleasant odours that can be detected in exhaled air. Halitosis is a condition that affects many people. There are multiple types of halitosis and they are classified depending on the source or cause of this bad breath: physiological halitosis, pathological halitosis (oral cause, 87% of all halitosis, and extraoral cause), pseudohalitosis and halitophobia. Bad breath through an oral cause is therefore the most common. This type of halitosis is caused by the production and release of volatile compounds, mainly sulphur. Other molecules such as the diamines (for example, cadaverine) are also related to halitosis. The volatile sulphur compounds are caused by bacteria that grow in the mouth in conditions of oxygen absence (anaerobes). These volatile sulphur compounds come from the metabolism of food bacteria or remain trapped in the mouth.

The term "caries" or "cavities" refers to the breakdown of teeth due to acids produced by bacteria. *Streptococcus mutans* is considered one of the main bacteria involved in this process. In addition, other microflora like *Candida* are also associated with active caries lesions [Prasanth M., Dent Res J (Isfahan), 2011, 8(2), 85-94]. The cavities may be a number of different colors from yellow to black. Symptoms may include pain and difficulty with eating. Complications may include inflammation of the tissue around the tooth, tooth loss, and infection or abscess formation. It is a disease characterized by a series of reactions that lead the teeth's hard tissues to soften, and which if it goes untreated, advances from the surface inwards, eventually leading to the irreversible destruction of the tooth. Caries normally goes unnoticed by the patient. A detailed exploration will detect stains or white chalk-like spots (areas where there is no enamel) or brown pigmentation on the tooth surface. This phase may be characterized by a certain sensitivity (strange sensation) to some foods, particularly sweets or hot or cold drinks, although usually there is no actual pain. When the caries advances and affects the pulp or nerve inside the tooth, pain will generally ensue, although if the affection is very slow it may destroy a large part of the nerve painlessly. When the affection is deep, abscesses may form (better known as gumboils, i.e. destroyed dental material and pus) and give rise to major inflammation and pain.

The term "peri-implant mucositis" refers to an inflammatory lesion of the soft tissues surrounding an endo-osseous implant in the absence of loss of supporting bone or continuing marginal bone loss. Peri-implant mucositis is

caused by bacteria from dental biofilms, which disrupts the host-microbe homeostasis at the implant-mucosa interface, resulting in an inflammatory lesion. Biofilm removal is a prerequisite for the prevention and management of peri-implant mucositis. Peri-implant mucositis is considered a precursor for peri-implantitis.

The term "periimplantitis" refers to an inflammatory lesion of the mucosa that affects the surrounding tissue of the implant, predominately the supporting bone with loss of osseointegration. As peri-implant mucositis, it is also caused by the adhesion of pathogenic bacterial biofilms on the implant surface and peri-implant tissues.

In the context of the invention, the term "about" when characterizing a value refers to ±10% of said value, preferably ±5% of said value.

Preferably, the expression "one or more" refers to 1, 2, 3, 4, 5, 6, 7, 8, 9 or 10, more preferably to 1, 2, 3, 4 or 5, even more preferably to 1, 2 or 3.

The following examples are provided to illustrate the invention but should not be considered as limiting its scope.

**Examples**

Compositions

Oral care compositions (450 g) were prepared by mixing the ingredients disclosed in the table below. The ingredients and their amounts (expressed as %w/w, i.e. wt%) of each composition is provided in the tables below.

| *Ingredient* | *Comparative example 1* | *Comparative example 2* | *Comparative example 3* |
|---|---|---|---|
| 4-Hydroxy acetophenone | 0.1 | 0.1 | 0.1 |
| Dipotassium glycyrrhizinate | 0 | 0 | 0.1 |
| *o*-Cymen-5-ol | 0 | 0.1 | 0 |
| Propylene glycol | 7 | 7 | 7 |
| PEG40 hydrogenated castor oil | 2 | 2 | 2 |
| Xanthan gum | 1 | 1 | 1 |
| Water | *q.s.* 100 | *q.s.* 100 | *q.s.* 100 |
| *Ingredient* | *Example 4* | *Example 5* | |
| 4-Hydroxy acetophenone | 0.1 | 0.1 | |
| Dipotassium glycyrrhizinate | 0.1 | 0 | |
| *o*-Cymen-5-ol | 0.1 | 0.1 | |
| Propylene glycol | 7 | 7 | |
| PEG40 hydrogenated castor oil | 2 | 2 | |
| Xanthan gum | 1 | 0 | |
| Water | *q.s.* 100 | *q.s.* 100 | |

The tables below disclose specific mouthwash, toothpaste and tooth gel formulations.

Mouthwash example formulation 6:

| *Ingredient* | *%w/w* |
|---|---|
| o-Cymen-5-ol | 0.10 |
| Dipotassium glycyrrhizinate | 0.05 |
| 4-Hydroxyacetophenone | 0.50 |
| Sodium fluoride | 0.31 |
| Potassium nitrate | 3.00 |
| Glycerine | 3.00 |
| Propylene glycol | 7.00 |

(continued)

| *Ingredient* | *%w/w* |
|---|---|
| PEG 40 Hydr. Castor oil | 2.00 |
| Panthenol | 0.25 |
| Chelating agents | 1.00 |
| Oral care active ingredient that inhibits plaque formation | 0.10 |
| Sweeterners | 1.00 |
| Binder | 0.20 |
| Flavoring agent | 0.20 |
| Water | 81.29 |
| Total | 100.00 |

Mouthwash example formulation 7:

| *Ingredient* | *%w/w* |
|---|---|
| o-Cymen-5-ol | 0.10 |
| Dipotassium glycyrrhizinate | 0.10 |
| 4-Hydroxyacetophenone | 0.50 |
| Sodium fluoride | 0.31 |
| Glycerine | 3.00 |
| Propylene glycol | 7.00 |
| PEG 40 Hydr. Castor oil | 2.00 |
| Panthenol | 0.25 |
| Chelating agents | 1.00 |
| Oral care active ingredient that inhibits plaque formation | 0.10 |
| Sweeterners | 1.00 |
| Binder | 0.20 |
| Flavoring agent | 0.20 |
| Water | 84.24 |
| Total | 100.00 |

Toothpaste example formulation 8:

| *Ingredient* | *%w/w* |
|---|---|
| o-Cymen-5-ol | 0.10 |
| Dipotassium glycyrrhizinate | 0.20 |
| 4-Hydroxyacetophenone | 0.20 |
| Oral care active ingredient that inhibits plaque formation | 0.60 |
| Sodium fluoride | 0.31 |
| Sorbitol | 43.00 |
| Glycerine | 12.00 |

(continued)

| *Ingredient* | %*w*/*w* |
|---|---|
| Propylene glycol | 2.00 |
| Silicates | 18.50 |
| Titanium dioxide | 1.00 |
| Surfactants | 3.50 |
| Sweeterners | 1.00 |
| Binder | 0.80 |
| Flavoring agent | 1.00 |
| Water | 15.79 |
| Total | 100.00 |

Tooth gel example formulation 9:

| *Ingredient* | %*w*/*w* |
|---|---|
| o-Cymen-5-ol | 0.10 |
| Dipotassium glycyrrhizinate | 0.20 |
| 4-Hydroxyacetophenone | 0.20 |
| Oral care active ingredient that inhibits plaque formation | 0.60 |
| Sodium fluoride | 0.31 |
| Sorbitol | 43.00 |
| Glycerine | 12.00 |
| Propylene glycol | 2.00 |
| Silicates | 18.50 |
| Surfactants | 3.50 |
| Sweeterners | 1.00 |
| Binder | 0.80 |
| Flavoring agent | 1.00 |
| Water | 16.79 |
| Total | 100.00 |

The binder is preferably xantan gum.

Rheological studies

Flow curves representing the change in the shear stress ($\tau$ in Pa) and the viscosity ($\eta$ in Pa·s) vs shear rate ($\dot{\gamma}$ in $s^{-1}$) for compositions of comparative examples 1-3 and for example 4 have been obtained in a rheometer (HAAKE MARS) by subjecting the compositions to the following conditions: shear rate from 0 to 100 $s^{-1}$ in 3 min, 1 min at a shear rate of 100 $s^{-1}$, and share rate from 100 to 0 $s^{-1}$ in 3 min. The yield stress ($\tau_0$) corresponds to the first values measured by the device when increasing shear rate from 0 to 100 $s^{-1}$ in 3 min. All rheological studies were conducted at a temperature of 20 °C. The experiments are carried out by applying 2 g of the test formulation to the sample area of the rheometer and, after lowering the movable part of the rheometer onto the sample area, excess formulation is removed.

The obtained flow curves were fitted to the Herschel-Bulkley model according to equation 1 (which describes a plastic fluid behavior) and to the Cross model according to equation 2 (which describes a pseudoplastic fluid behavior) [Alderman N. (1997), "Non-Newtonian Fluids: Guide to Classification and Characteristics", ESDU data Item 97034].

Herschel-Bulkley model:

$$\tau = \tau_0 + K\dot{\gamma}^n \qquad \textit{(Equation 1)}$$

where
$\tau$ is the shear stress (in Pa),
$\tau_0$ is the yield stress (in Pa),
$K$ is a coefficient (in Pa·s$^n$),
$\dot{\gamma}$ is the shear rate (in s$^{-1}$), and
$n$ is the exponent.
Cross model:

$$\eta = \eta_\infty + \frac{\eta_0 - \eta_\infty}{1 + (k\dot{\gamma})^n}$$

where
$\eta$ is the dynamic viscosity (in Pa·s),
$\eta_0$ is the asymptotic value of viscosity at very low shear rate (in Pa·s),
$\eta_\infty$ is the asymptotic value of viscosity at very high shear rate (in Pa·s),
$k$ is a coefficient (in s$^n$),
$\dot{\gamma}$ is the shear rate (in s$^{-1}$), and
$n$ is the exponent.

The correlation coefficient (r) of the fitting and corresponding equation parameters were calculated. The model having the highest correlation coefficient was selected.

The results are presented in the table below:

| *Composition* | *Fitting to Herschel-Bulkley model* | *Fitting to Cross model* |
|---|---|---|
| Comparative example 1 | $\tau_0$ = 3.146 Pa<br>$K$ = 11.6Pa·s$^n$<br>$n$ = 0.08037<br>$r$ = 0.9847 | $\eta_0$ = 17.12 Pa·s<br>$\eta_\infty$ = 0.07059 Pa·s<br>$\dot{\gamma}$ = 1.454 s$^{-1}$<br>*n = 1.137*<br>$r$ = 0.866 |
| Comparative example 2 | $\tau_0$ = 3.396 Pa<br>$K$ = 14.59·s$^n$<br>$n$ = 0.0867<br>$r$ = 0.9879 | $\eta_0$ = 20.46 Pa·s<br>$\eta_\infty$ = 0.08067 Pa·s<br>$\dot{\gamma}$ = 1.443 s$^{-1}$<br>$n$ = 1.113<br>$r$ = 0.8488 |
| Comparative example 3 | $\tau_0$ = 3.291 Pa<br>$K$ = 9.602·s$^n$<br>$n$ = 0.1018<br>$r$ = 0.9807 | $\eta_0$ = 15.09 Pa·s<br>$\eta_\infty$ = 0.0679 Pa·s<br>$\dot{\gamma}$ = 1.447 s$^{-1}$<br>$n$ = 1.124<br>$r$ = 0.8411 |
| Example 4 | $\tau_0$ = -321.5 Pa<br>$K$ = 332.9·s$^n$<br>$n$ = 0.004342<br>$r$ = 0.9628 | $\eta_0$ = 332.5 Pa·s<br>$\eta_\infty$ = 0.03132 Pa·s<br>$\dot{\gamma}$ = 0.04075 s$^{-1}$<br>$n$ = 0.9833<br>$r$ = 0.9942 |

These results gathered in the table above show that the compositions of comparative examples 1-3 do fit very well to the Herschel-Bulkley model, i.e. these compositions have a plastic fluid behavior, whereas the composition of

example 4 fits very well to the Cross model, i.e. it has a pseudoplastic behavior.

The yield stress $\tau_0$ was determined for the compositions in order to verify the above results. The obtained experimental values are provided in the table below:

| *Composition* | $\tau_0$ |
|---|---|
| Comparative example 1 | 3.1 Pa |
| Comparative example 2 | 3.3 Pa |
| Comparative example 3 | 3.1 Pa |
| Example 4 | 0 Pa |

These results confirm that the composition of Example 4 has a pseudoplastic behavior since it has no yield stress ($\tau_0$ = 0 Pa), whereas compositions of comparative examples 1-4 have yield stress values in the range of 3.1-3.3 Pa.

Microbiological studies

Studies of antimicrobial activity against *Streptococcus mutans* (ATCC 25175) and *Candida albicans* (ATCC 90028) were carried out with the compositions of Example 4. The control was a composition comprising 2 wt% of PEG 40 hydrogenated castor oil, 7 wt% propylene glycol and water (*q.s.* 100 wt%).

Culture media and incubation conditions for each microorganism are provided in the table below:

| *Microorganism* | *Culture media* | *Incubation conditions* |
|---|---|---|
| *S. mutans* | Trypticase Soy Agar (TSA)/ Trypticase Soy Broth (TSB)/ Müller-Hinton Broth | 37 °C, 5% $CO_2$, 48 h |
| *C. albicans* | Trypticase Soy Broth (TSB)/ Sabouraud-Cloramfenicol/ RPMI 1640 + 0.2% glucose | 30 °C, aerobiosis, 24-48 h |

Determination of maximum inhibitory dilution (MID):

Various dilutions of the compositions under study were incubated with the microorganisms ($10^4$ cfu/mL) cultured in microtiter plates in the corresponding culture media and incubation conditions. The MID was defined as the highest dilution (i.e. lowest concentration) capable of totally inhibiting visible microbial growth. [EUCAST (European Committee on Antimicrobial Susceptibility Testing) and CLSI (Clinical and Laboratory Standards Institute) guidelines]

Determination of maximum bactericidal dilution (MBD):

20 μL of the dilutions of the compositions under study from the MID experiment described above where no visible microorganism growth had been observed in the MID assay were seeded in a solid agar medium. The seeded plates were incubated under the conditions described in the table above. The MBD corresponded to the highest dilution (i.e. lowest concentration) capable of totally inhibiting microbial growth.

Determination of bacterial death kinetics:

Bacterial death kinetics of the compositions under study was determined by contacting suspensions of bacteria at a concentration of from $5\times10^5$ to $2\times10^6$ cfu/mL. After 1, 2 and 5 minutes a sample (100 μL) was extracted and diluted 100 times. The number of viable cells was determined by seeding in solid agar medium. The plates were incubated under the corresponding conditions provided in the table above and the number of bacteria was determined.

The results of MID and MBD are provided in the Table below, expressed as the dilution of the composition tested.

| | *S. mutans* | | *C. albicans* | |
|---|---|---|---|---|
| | MID | MBD | MID | MBD |
| Control | ≥ 1/2 | ≥ 1/2 | ≥ 1/2 | ≥ 1/2 |
| Example 4 | 1/2 | ≥ 1/2 | 1/2 | ≥ 1/2 |

The composition of Example 4 showed antibacterial activity against *S. mutans* and C. *albicans.*

The composition of Example 4 was further tested to determine its bacterial death kinetics against C. *albicans.* The results are provided in the Table below expressed as bacterial counts (cfu/mL) and % of survival in parentheses.

| | Bacterial counts (% survival) |
|---|---|
| 0 min | $5.00 \cdot 10^5$ (100%) |
| 1 min | $6.00 \cdot 10^4$ (12%) |
| 2 min | $1.20 \cdot 10^4$ (2.40%) |
| 5 min | $2.50 \cdot 10^4$ (5%) |

The composition of Example 4 showed bacteriostatic activity against C. *albicans* and is a fast active composition against this type of bacteria.

## Claims

1. Oral care composition comprising:

   - 0.005 to 1 wt% of o-cymen-5-ol,
   - 0.005 to 1.5 wt% of dipotassium glycyrrhizinate,
   - 0.05 to 5 wt% of 4-hydroxyacetophenone, and
   - at least 15 wt% of water,

   wherein the wt% are expressed with respect to the total weight of the composition.

2. Oral care composition according to claim 1, wherein water is present in an amount of at least 20 wt%, at least 25 wt%, at least 30 wt%, at least 40 wt%, at least 50 wt%, at least 60 wt%, at least 70 wt%, at least 75 wt%, or at least 80 wt%, wherein the wt% is expressed with respect to the total weight of the composition.

3. Oral care composition according to any of the preceding claims further comprising one or more ingredients selected from the group consisting of fluoride ion sources, surfactants, co-solvents, humectants, sweeteners, binders, mouth-feel agents, abrasives, desensitizing agents, other oral care active ingredients, pH modifying agents, preservatives, chelating agents, flavoring agents, and colorants.

4. Oral care composition according to any of the preceding claims in the form of a mouthwash, a tooth gel or a toothpaste.

5. Oral care composition according to any of the preceding claims in the form of a mouthwash which comprises:

   - 0.05 to 1.5 wt% of 4-hydroxyacetophenone,
   - 0.005 to 1 wt% of o-cymen-5-ol,
   - 0.005 to 1.5 wt% of dipotassium glycyrrhizinate,
   - at least 70 wt% of water,
   - optionally 5 to 10 wt% of propylene glycol,
   - optionally 1 to 10 wt% of glycerin, and
   - optionally 1 to 5 wt% PEG40 hydrogenated castor oil,

   wherein the wt% is expressed with respect to the total weight of the composition.

6. Oral care composition according to any of claims 1 to 4 in the form of a tooth gel or toothpaste which comprises:

   - 0.05 to 1.5 wt% of 4-hydroxyacetophenone,
   - 0.005 to 1 wt% of o-cymen-5-ol,
   - 0.005 to 1.5 wt% of dipotassium glycyrrhizinate,

- 15 to 35 wt% of water,
- 30 to 55 wt% of sorbitol,
- optionally 0.1 to 1.5 wt% xanthan gum,
- optionally 0.5 to 10 wt% of propylene glycol, and
- optionally 1 to 15 wt% of glycerin,

wherein the wt% is expressed with respect to the total weight of the composition.

**7.** Oral care composition according to any of the preceding claims, wherein the weight ratio of 4-hydroxyacetophenone to dipotassium glycyrrhizinate is from 15:1 to 0.5:1.

**8.** Oral care composition according to any of the preceding claims, wherein the weight ratio of 4-hydroxyacetophenone to the o-cymen-5-ol is from 10:1 to 0.5:1.

**9.** Oral care composition according to any of the preceding claims for use in the treatment and/or prevention of dental plaque formation, caries formation, calculus formation, halitosis, gingivitis, periodontitis, peri-implant mucositis and/or periimplantitis.

**Patentansprüche**

**1.** Mundpflegezusammensetzung, umfassend:

- 0,005 bis 1 Gew.% *o*-Cymen-5-ol,
- 0,005 bis 1,5 Gew.% Dikaliumglycyrrhizinat,
- 0,05 bis 5 Gew.% 4-Hydroxyacetophenon, und
- wenigstens 15 Gew.% Wasser,
- wobei die Gew.%-Angaben in Bezug auf das Gesamtgewicht der Zusammensetzung ausgedrückt sind.

**2.** Mundpflegezusammensetzung nach Anspruch 1, wobei Wasser in einer Menge von wenigstens 20 Gew.%, wenigstens 25 Gew.%, wenigstens 30 Gew.%, wenigstens 40 Gew.%, wenigstens 50 Gew.%, wenigstens 60 Gew.%, wenigstens 70 Gew.%, wenigstens 75 Gew.%, oder wenigstens 80 Gew.% vorliegt, wobei die Gew.%-Angabe in Bezug auf das Gesamtgewicht der Zusammensetzung ausgedrückt ist.

**3.** Mundpflegezusammensetzung nach einem der vorangehenden Ansprüche, weiterhin umfassend einen oder mehrere Inhaltsstoffe, ausgewählt aus der Gruppe, bestehend aus Fluoridionen-Quellen, oberflächenaktiven Mitteln, Co-Lösungsmitteln, Feuchthaltemitteln, Süßstoffen, Bindemitteln, Mundgefühlmitteln, Schleifmitteln, Desensibilisierungsmitteln, anderen aktiven Inhaltsstoffen für die Mundpflege, pH-Modifizierungsmitteln, Konservierungsmitteln, chelatbildenden Mitteln, Geschmacksstoffen und Farbstoffen.

**4.** Mundpflegezusammensetzung nach einem der vorangehenden Ansprüche, in der Form einer Mundspülung, eines Zahngels oder einer Zahnpasta.

**5.** Mundpflegezusammensetzung nach einem der vorangehenden Ansprüche, in der Form einer Mundspülung, die umfasst:

- 0,05 bis 1,5 Gew.% 4-Hydroxyacetophenon,
- 0,005 bis 1 Gew.% *o*-Cymen-5-ol,
- 0,005 bis 1,5 Gew.% Dikaliumglycyrrhizinat
- wenigstens 70 Gew.% Wasser,
- wahlweise 5 bis 10 Gew.% Propylenglykol,
- wahlweise 1 bis 10 Gew.% Glycerin, und
- wahlweise 1 bis 5 Gew.% PEG40-hydriertes Rizinusöl,
- wobei die Gew.%-Angabe in Bezug auf das Gesamtgewicht der Zusammensetzung ausgedrückt ist.

**6.** Mundpflegezusammensetzung nach einem der Ansprüche 1 - 4, in der Form eines Zahngels oder Zahnpasta, die umfasst:

- 0,05 bis 1,5 Gew.% 4-Hydroxyacetophenon,
- 0,005 bis 1 Gew.% *o*-Cymen-5-ol,
- 0,005 bis 1,5 Gew.% Dikaliumglycyrrhizinat,
- 15 bis 35 Gew.% Wasser,
- 30 bis 55 Gew.% Sorbitol,
- wahlweise 0,1 bis 1,5 Gew.% Xanthangummi,
- wahlweise 0,5 bis 10 Gew.% Propylenglykol, und
- wahlweise 1 bis 15 Gew.% Glycerin,

wobei die Gew.%-Angabe in Bezug auf das Gesamtgewicht der Zusammensetzung ausgedrückt ist.

**7.** Mundpflegezusammensetzung nach einem der vorangehenden Ansprüche, wobei das Gewichtsverhältnis von 4-Hydroxyacetophenon zu Dikaliumglycyrrhizinat von 15:1 bis 0,5:1 beträgt.

**8.** Mundpflegezusammensetzung nach einem der vorangehenden Ansprüche, wobei das Gewichtsverhältnis von 4-Hydroxyacetophenon zu dem *o*-Cymen-5-ol von 10:1 bis 0,5:1 beträgt.

**9.** Mundpflegezusammensetzung nach einem der vorangehenden Ansprüche, zur Verwendung bei der Behandlung und/oder Verhinderung von Zahnbelagbildung, Kariesbildung, Zahnsteinbildung, Mundgeruch, Gingivitis, Parodontitis, periimplantäre Mukositis und/oder Periimplantitis.

**Revendications**

**1.** Composition d'hygiène buccale comprenant :

- de 0,005 à 1 % en poids d'o-cymén-5-ol,
- de 0,005 à 1,5 % en poids de glycyrrhizinate dipotassique,
- de 0,05 à 5 % en poids de 4-hydroxyacétophénone, et
- au moins 15 % en poids d'eau,

les % en poids étant exprimés par rapport au poids total de la composition.

**2.** Composition d'hygiène buccale selon la revendication 1, dans laquelle l'eau est présente en une quantité d'au moins 20 % en poids, au moins 25 % en poids, au moins 30 % en poids, au moins 40 % en poids, au moins 50 % en poids, au moins 60 % en poids, au moins 70 % en poids, au moins 75 % en poids ou au moins 80 % en poids, le % en poids étant exprimé par rapport au poids total de la composition.

**3.** Composition d'hygiène buccale selon l'une quelconque des revendications précédentes comprenant en outre un ou plusieurs ingrédients choisis dans le groupe constitué par des sources d'ions fluorure, des tensio-actifs, des co-solvants, des humectants, des édulcorants, des liants, des agents de sensation en bouche, des abrasifs, des agents désensibilisants, d'autres ingrédients actifs d'hygiène buccale, des agents modifiant le pH, des conservateurs, des agents chélateurs, des agents aromatisants et des colorants.

**4.** Composition d'hygiène buccale selon l'une quelconque des revendications précédentes sous forme d'un bain de bouche, d'un gel dentaire ou d'un dentifrice.

**5.** Composition d'hygiène buccale selon l'une quelconque des revendications précédentes sous forme de bain de bouche, qui comprend :

- de 0,05 à 1,5 % en poids de 4-hydroxyacétophénone,
- de 0,005 à 1 % en poids d'o-cymén-5-ol,
- de 0,005 à 1,5 % en poids de glycyrrhizinate dipotassique,
- au moins 70 % en poids d'eau,
- optionnellement, de 5 à 10 % en poids de propylène glycol,
- optionnellement, de 1 à 10 % en poids de glycérine, et
- optionnellement, de 1 à 5 % en poids d'huile de ricin hydrogénée PEG40,

le % en poids étant exprimé par rapport au poids total de la composition.

6. Composition d'hygiène buccale selon l'une quelconque des revendications 1 à 4 sous forme de gel dentaire ou de dentifrice qui comprend :

   - de 0,05 à 1,5 % en poids de 4-hydroxyacétophénone,
   - de 0,005 à 1 % en poids d'o-cymén-5-ol,
   - de 0,005 à 1,5 % en poids de glycyrrhizinate dipotassique,
   - de 15 à 35 % en poids d'eau,
   - de 30 à 55 % en poids de sorbitol,
   - optionnellement, de 0,1 à 1,5 % en poids de gomme xanthane,
   - optionnellement, de 0,5 à 10 % en poids de propylène glycol, et
   - optionnellement, de 1 à 15 % en poids de glycérine,

   le % en poids étant exprimé par rapport au poids total de la composition.

7. Composition d'hygiène buccale selon l'une quelconque des revendications précédentes, dans laquelle le rapport pondéral de la 4-hydroxyacétophénone au glycyrrhizinate dipotassique est de 15:1 à 0,5:1.

8. Composition d'hygiène buccale selon l'une quelconque des revendications précédentes, dans laquelle le rapport pondéral de la 4-hydroxyacétophénone à l'o-cymén-5-ol est de 10:1 à 0,5:1.

9. Composition d'hygiène buccale selon l'une quelconque des revendications précédentes destinée à être utilisée dans le traitement et/ou la prévention de la formation de plaque dentaire, de la formation de caries, de la formation de tartre, de l'halitose, de la gingivite, de la parodontite, de la mucite péri-implantaire et/ou de la péri-implantite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ASL, M. N. et al.** *Phytother. Res.,* 2008, vol. 22, 709-724
- **PARK, Y.-D. et al.** *Int. J. Clin. Prev. Dent.,* 2010, vol. 6 (2), 55-61
- **CHO et al.** *Int J Oral Health,* 2009, vol. 5, 37-47
- **PARK et al.** *Int. J. Clin. Prev. Dent.,* 2010, vol. 6 (2), 55-61
- **MITSOULIS.** *J. Non-Newtonian FluidMech.,* 2007, vol. 141, 138-147
- **PRASANTH M.** *Dent Res J (Isfahan),* 2011, vol. 8 (2), 85-94
- **MENENDEZ A. et al.** *Oral Microbiology Immunology,* 2005, vol. 20 (1), 31-34
- **ALDERMAN N.** *Non-Newtonian Fluids: Guide to Classification and Characteristics,* 1997